(19) Europäisches Patentamt
European Patent Office
Office européen des brevets

(11) **EP 2 969 995 B1**

(12) **EUROPEAN PATENT SPECIFICATION**

(45) Date of publication and mention
of the grant of the patent:
**06.01.2021 Bulletin 2021/01**

(21) Application number: **14722333.3**

(22) Date of filing: **14.03.2014**

(51) Int Cl.:
**C03C 23/00** (2006.01)     **C03B 23/045** (2006.01)
**C03B 23/057** (2006.01)     **C03B 23/09** (2006.01)
**G01N 33/487** (2006.01)

(86) International application number:
**PCT/IB2014/059789**

(87) International publication number:
**WO 2014/141168 (18.09.2014 Gazette 2014/38)**

(54) **MODIFICATION OF ORIFICES IN GLASS NANOCAPILLARIES**

MODIFIKATION VON ÖFFNUNGEN IN NANOKAPILLAREN AUS GLAS

MODIFICATIONS D'ORIFICES DANS DES NANOCAPILLAIRES DE VERRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 PCT/IB2013/052093**

(43) Date of publication of application:
**20.01.2016 Bulletin 2016/03**

(73) Proprietor: **École Polytechnique Fédérale de Lausanne (EPFL)**
**1015 Lausanne (CH)**

(72) Inventors:
• **STEINBOCK, Lorenz Jan**
**CH-1015 Chavannes-près-Renens (CH)**
• **RADENOVIC, Aleksandra**
**CH-1025 St-Sulpice (CH)**

(74) Representative: **Grosfillier, Philippe**
**ANDRE ROLAND S.A.**
**IP Attorneys & Services**
**Avenue Collonges 21**
**1004 Lausanne (CH)**

(56) References cited:
**WO-A1-00/34810          US-A1- 2006 231 774**
**US-A1- 2014 103 582**

• **BO ZHANG ET AL: "A Silica Nanochannel and Its Applications in Sensing and Molecular Transport", ANALYTICAL CHEMISTRY, vol. 81, no. 13, 1 July 2009 (2009-07-01), pages 5541-5548, XP055123300, ISSN: 0003-2700, DOI: 10.1021/ac9009148**
• **STEINBOCK L J ET AL: "Probing DNA with micro-Â and nanocapillaries and optical tweezers;Probing DNA with micro-and nanocapillaries and optical tweezers", JOURNAL OF PHYSICS: CONDENSED MATTER, INSTITUTE OF PHYSICS PUBLISHING, BRISTOL, GB, vol. 22, no. 45, 29 October 2010 (2010-10-29), page 454113, XP020199821, ISSN: 0953-8984, DOI: 10.1088/0953-8984/22/45/454113**
• **ANMIV S PRABHU ET AL: "SEM-induced shrinking of solid-state nanopores for single molecule detection;SEM-induced shrinking of solid-state nanopores for single molecule detection", NANOTECHNOLOGY, IOP, BRISTOL, GB, vol. 22, no. 42, 22 September 2011 (2011-09-22), page 425302, XP020211985, ISSN: 0957-4484, DOI: 10.1088/0957-4484/22/42/425302**
• **LIEBERMAN K ET AL: "Multifunctional, micropipette based force cantilevers for scanned probe microscopy", APPLIED PHYSICS LETTERS, AMERICAN INSTITUTE OF PHYSICS, US, vol. 65, no. 5, 1 August 1994 (1994-08-01), pages 648-650, XP002329223, ISSN: 0003-6951, DOI: 10.1063/1.112259**
• **VEIKO, V.P.: "Combined Nanoprobes for Scanning Probe Microscopy", SPIE, PO BOX 10 BELLINGHAM WA 98227-0010 USA, 1 January 2008 (2008-01-01), XP040433707,**

**Description**

**Technical field**

The invention relates to the manufacturing of small size orifices in glass-like materials.

**State-of-the-art**

When dealing with nanotechnology, e.g. when the dimensions of an orifice only have a few nanometers, specific effects may arise. Especially the resistive pulse technique (also called Coulter counter technique) may benefit from this technique. It is a versatile method to count and characterize single cells, colloids, molecules or even sequence DNA[1-3]. An electric potential is applied through a small orifice incorporated into a membrane or cone. This potential creates an ionic current whose amplitude depends, besides parameters such as ionic concentration and surface charge, on the volume and size of the orifice[4]. Orifices with diameters in the nanometer range are named nanopores, which are divided into solid-state and biological nanopores[5]. The smaller this nanopore is, the bigger is the current blockade from translocating molecules, which increases the signal-to-noise ratio.

Classical solid-state nanopores have seen a large variety of different techniques emerging in the last decade to decrease their size[6-11]. Smaller diameters are favorable since they permit to detect smaller molecules because the current change due to the analyte entering the nanopore increases with smaller nanopores[12]. First experiments were performed by Storm et al. who shrunk nanopores in silicon oxide from 40 to about 3 nm in diameter using a transmission electron microscopy (TEM) beam[13,14]. The shrinking was explained by the surface stress causing the fluidized silicon oxide to contract in order to minimize the surface of the nanopore. This was followed by experiments where scanning electron microscopes (SEM) were used to either shrink nanopores by silicon oxide deposition[15] or thermal induced shrinking[16,17]. The importance of thermal heating was recently demonstrated by Asghar et al. who caused nanopores to decrease in diameter by heating them up in an oven to about 1000 degree Celsius[18]. Since carbon deposition is often observed under SEM irradiation nanopore shrinking was also investigated with energy-dispersive X-ray spectroscopy (EDX) by Prabhu et al. who could rule out significant carbon deposition[19,20]. The shrink rates were also examined as a function of the SEM magnification showing a positive linear response to the magnification[19]. In contrast a power law dependence was observed for the beam potential (also called extra-high tension, EHT), where smaller shrink rates were observed at higher voltages[19,21]. This was supported by Monte-Carlo simulations showing increased penetration depths for electrons at higher beam potentials. The higher penetration depths cause smaller energy densities and hence less thermal heating leading to smaller shrink rates. Both shrinking techniques with a TEM or SEM have the advantage to allow live observation of the shrinking process. This permits to stop the shrinking process at any desired size. A new approach was pursued by Ayub et al. who electrodeposited Pt on the planar membrane nanopore interface while already immersed in the ionic solution[22]. The electrodeposition process is monitored in situ and can be stopped at any desired conductance level. Another technique is atomic layer deposition (ALD), which deposits angstrom thick layers of i.e. aluminum oxide to decrease the nanopore size[23,24]. Compared to electronic microscopy shrinking, above mentioned methods do not permit to see the size of the nanopore but to deduce the diameter from the conductance.

All these previous experiments focused on planar membranes made out of silicon-like materials fabricated in complementary metal-oxide-semiconductor (CMOS) techniques such as etching and vapor deposition. Especially invention WO 2004/078640 focusses on several occasions on the use of membranes (page 3 line 17, page 6 line 25, page 7 line 29, page 8 line 5 and 17). The disadvantage of using planar orifices in membranes is that they cannot be approached to a surface in the distance of a few nanometers. Due to roughness of the membrane or of the surface itself this will cause the breaking of the thin and fragile membrane. For this purpose conical shaped orifices from pipette pulling machines are used since several years in application like patch clamping (also called stochastic sensind), scanning ion conductance microscopy (SICM), scanning electrochemical microscopy (SECM) or near-field scanning optical microscopy (NSOM). There the conical orifice has to be approached to a cell surface or substrate surface and be robust enough to not break if touching the substrate. The use of nanopores in planar membranes instead would have not been possible. A helping comparison is the necessity of using a sharp tip when revealing the topography of a surface with an atomic force microscopy (AFM). A flat tip, which is like a big membrane would only result in bad resolved images of the surface. Very similar to this comparison is the difference between an orifice in a membrane to an orifice in a cone leading to different uses of this completely different geometries.

WO0034810 discloses a method for producing subwavelength near-field optical apertures includes directing a laser beam at the top of a tapered optical fiber to melt the fiber tip. The melting forms a lens at the tip, and the resulting structure is then coated with metal in such a way that an aperture is left open, or the aperture is formed in the metal by means of an ion beam or a laser beam. In another embodiment, the tip of a micropipette is melted by a laser beam to form an aperture having thick walls.

The article entitled "A Silica Nanochannel and Its Applications in Sensing and Molecular Transport", by BO ZHANG ET AL and published in ANALYTICAL CHEMISTRY, (20090701), vol. 81, no. 13, doi:10.1021/ac9009148, ISSN 0003-2700, pages 5541 - 5548 discloses the preparation, characterization, and analytical application of silica nanochannels in the size range of 5-100 nm. These cylindrical-shaped nanochannels are prepared using a simple laser-assisted mechanical pulling process, followed by partial enclosure into a glass micropipet. The nanochannels are characterized using a combination of optical microscopy, scanning electron microscopy (SEM), and resistance measurements in an electrolyte solution. The SEM results show that the nanochannel has circular geometry at the orifice. Ohmic response has been obtained from current-voltage measurements in KCl solutions using a silica nanochannel as small as 9 nm in diameter. These nanochannels have been utilized to sense single 40 nm polystyrene nanoparticles. A linear response has been observed between the detection rate and the concentration of nanoparticles in the range of 0-25 nM. The silica nanochannels have also been applied to the study of molecular transport of double-stranded DNA. Electroosmosis-driven molecular translocation has been observed for genomic-length A-DNA through a 9 nm nanochannel in a 3M KCl solution.

The article entitled "Probing DNA with micro-and nanocapillaries and optical tweezers" by STEINBOCK L J ET AL, published in JOURNAL OF PHYSICS: CONDENSED MATTER, INSTITUTE OF PHYSICS PUBLISHING, BRISTOL, GB, (20101029), vol. 22, no. 45, doi:10.1088/0953-8984/22/45/454113, ISSN 0953-8984, page 454113 disclose optical tweezer experiments with the resistive pulse technique based on capillaries. Quartz glass capillaries are pulled into a conical shape with tip diameters as small as 27 nm. Here, it is discussed the translocation of A-phage DNA which is driven by an electrophoretic force through the nanocapillary. The resulting change in ionic current indicates the folding state of single A-phage DNA molecules. Our flow cell design allows for the straightforward incorporation of optical tweezers. It is shown that a DNA molecule attached to an optically trapped colloid is pulled into a capillary by electrophoretic forces. The detected electrophoretic force is in good agreement with measurements in solid-state nanopores.

WO2004078640 discloses a method for manufacturing nanometer-scale apertures, wherein, in an object, in a conventional manner, at least one aperture is provided with a nanometer-scale surface area, after which, by means of an electron beam, energy is supplied to at least the edge of said at least one aperture, such that the surface area of the respective aperture is adjusted, wherein the surface area of the aperture is controlled during adjustment and the supply of energy is regulated on the basis of the surface area change.

The article entitled "SEM-induced shrinking of solid-state nanopores for single molecule detection" by ANMIV S PRABHU ET AL, and published in NANOTECHNOLOGY, IOP, BRISTOL, GB, (20110922), vol. 22, no. 42, doi:10.1088/0957-4484/22/42/425302, ISSN 0957-4484, page 425302 discloses a mechanism by which the diameter of solid-state nanopores is reduced by a scanning electron microscope. The process depends on beam parameters such as the accelerating voltage and electron flux and does not involve simple electron-beam-induced deposition of hydrocarbon contaminants. Instead, it is an energy-dependent process that involves material flow along the surface of the nanopore membrane. It is also shown that pores fabricated in this manner can detect double stranded DNA.

**General description of the invention**

The present invention concerns a process according to claim 1.

The present invention relates to the manufacturing of small size orifices in glass-like materials wherein said material has a tubular shape, for instance a conical tubular shape. The invention is used with nanocapillaries. A nanocapillary is a glass cone with the
orifice at its tip. This structure is very different from an orifice in a CMOS fabricated planar membrane and is made using a so called pipette puller. The pipette puller was originally fabricated for patch and voltage clamping techniques. A laser heats a hollow glass capillary in the middle. The glass can be made out of element, which becomes viscous when heated and becomes solid when cooled down to temperature below 100 degrees. Exemplary elements and alloys are silicon, oxide, aluminum, metals, steel and titanium. Two clamps pull from both sides of the capillary. The heated spot elongates and shrinks in its diameter until a point where the shrunken and heated part breaks and the capillary separates into two capillaries. These two capillaries have a conical shape with an orifice at its tip. Until now imaging of these nanometer sized tip with an SEM were only possible when the tips were coated with a conducting material such as gold or platinum[25]. However due to improved scanning electrons microscopy detectors uncoated glass capillaries, could be imaged under an SEM. While doing these image recordings the inventors accidentally realized that shape changing orifice was not caused by charging effects by the electron beam but by the heating of the electron beam. Such an effect has never been reported for conical glass capillaries until now.

The area of this orifice can be shrunken under irradiation such as electron radiation. Increasing the current of irradiation increases the shrink rate, increasing the acceleration potential of the irradiating particles reduces the shrink rate. Imaging the orifice with an electron microscope allows to see the shrinking process and stop at the desired size. Furthermore, the shrink rate can be fine-tuned by changing the current, magnification or acceleration potential.

Other applications which would benefit from controllable orifice shrinking of glass nanocapillaries include for example near-field scanning optical microscopy, scanning ion conductance microscopy, scanning electrochemical microscopy, glass nanopore membranes, micro electrode cavity arrays, electron spray techniques, mass spectroscopy, surface Raman spectroscopy, patch clamping, plasma physics, fluorescent detection of molecules translocating through nanocapillaries, filter techniques, 3D and 2D printing techniques, capillary electrophoresis, combination of nanocapillaries with optical tweezers, cell surgery for sample injection or removal, highly charged ion physics for plasma analysis, dark field microscopy and soft x-ray scanning microscopy.

Besides solid-state nanopores in silicon dioxide, silicon nitride or metal membranes, glass nanocapillaries have emerged in the last years as a cost-effective and versatile source of nanopores for single molecule detection[25]. They are fabricated using a laser pipette puller and not classical etch and evaporation techniques used in CMOS production lines. The pipette puller heats the cylindrical hollow capillary and stretches it at the same time. This causes the glass capillary to shrink in diameter at the heated spot and finally break into two conical tips defined as nanocapillaries. Depending on the parameters used during the fabrication such as pull strength or heat different end diameters can be reached ranging from micro- to tens of nanometers[26,27]. This wide range of end diameters, a fast, cheap and user-friendly fabrication process, and no need for clean rooms or TEMs is an advantage comparing it to the fabrication needs for other solid-state nanopores. Reliably diameters of 30 nm can be reached using laser pullers, however to increase the sensitivity for smaller molecules smaller diameters would be essential[28]. Hence, a technique would be needed to further decrease the diameter of the laser-pulled glass nanocapillaries.

Glass nanocapillaries fabricated directly from a laser pipette puller, have not yet benefited from a shrinking method with live optical monitoring of the shrinking nanocapillary. Quartz Nanopore Membrane (QNM) require the incorporation of a sharp polished Pt wire into a glass membrane, which is etched away while monitoring the conductance of the opening nanopore[29]. A similar technique is used by Gao et al. who corrode a nanopore into a sealed capillary pipett using an HF solution[28]. Again the pore opening is controlled non-optically by measuring the conductance. A novel technique where the pore diameter can even be changed in situ was presented by Platt, Willmott and Lee who use a tunable pore made out of thermoplastic polyurethane membrane[30].

The invention covers a method to shrink glass (made out of quartz or borosilicate glass) nanocapillaries to any size from up to 200 nm to a few nanometers or even to the complete closure of the nanopore. The shrinking occurs while observing the glass nanocapillary under an SEM allowing precise control of their size by terminating the process at any desired diameter. The shrinking is fast reaching 0.25 nm/s and can be changed by adjusting the beam current or the electron acceleration voltage. This method has various applications like in the resistive pulse technique, where small diameters increase the sensing sensitivity. This is due to the increase in the amplitude of the current change, when the molecule enters the nanopore. In the future this will facilitate the detection of very small and short polymers[31]. The increase in the current amplitude will be demonstrated showing an increase from about 50 pA for single DNA strand for an unmodified nanocapillary to over 300 pA for a shrunken nanocapillary. Other applications which will benefit from a smaller diameter of the nanocapillary include near-field scanning optical microscopy (NSOM), scanning electrochemical microscopy (SECM), 2D/3D printing, scanning ion conductance microscopy or nanobilayer coated glass capillaries[32-34]. Advantages of this process are that the shrinking can be parallelized to multiple of cones by exposing them all to the heat or radiation source. Furthermore by use of the microscopy technique such as the electron microscope the shrinking can be observed live. Here the electron beam acts as a heating source as well as a mean to image the feature.

One of the important features of the invention is the shrinking of orifices in a tubular glass-like materials, eg a cone, from hundreds of nanometer in size to a few nanometers. The shrinking should be observable by microscopy technique such as an electron microscope. This allows stopping the shrinking at any desired feature size. The cone results from fabrication in a pipette puller designed to fabricate cones with orifices in glass capillaries and fibers.

Another important feature of the invention is the use of a radiation beam (made of particles and/or waves) to heat and then shrink the materials.

## Detailed description of the invention

The invention will be better understood below with some examples and some illustrations.

Of course the invention is not limited to those examples.

## Brief description of the figures

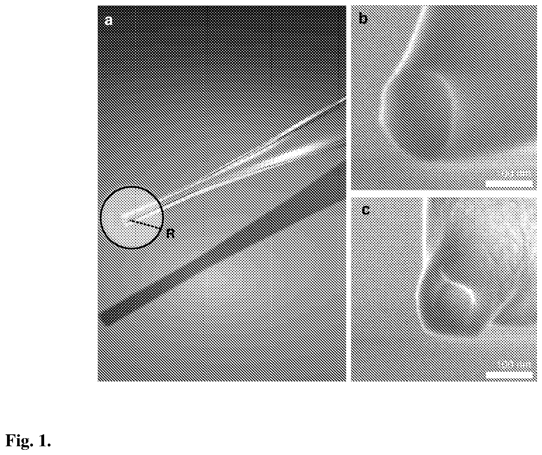

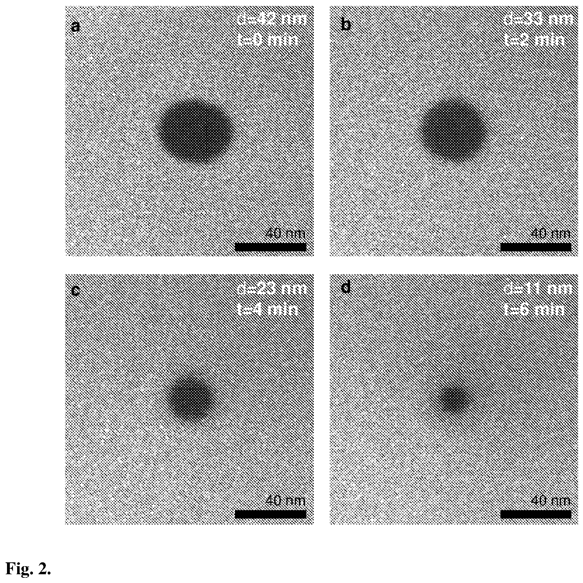

**Fig. 1.** (a) Scheme of the conical end of the nanocapillary. The shaded area depicts the region, which is imaged by the SEM beam. The Radius R is the penetration depth of the electron beam calculated by the theoretical penetration formula given by Kanaya-Okayama. (b) SEM in-lens image of a quartz nanocapillary magnified 196 kX times at a stage angle of 60 degrees to increase the three dimensional perception. The electron high tension was at 3.0 kV, the beam current was at 171 pA and the working distance was 3.3 mm. (c) Shrunken nanocapillary after 14 minutes of irradiation under constant angle and beam parameters. The process of deformation is also shown in a video in the SI (SI_movie_1.avi). Clearly a reshaping of the nanocapillary is visible precluding the possibility of significant carbon deposition. This was supported by EDX measurements included in the supplementary information in Figure 1., Figure 2. and Table S1.

**Fig. 2.** (a) Top side SEM in-lens image of a nanocapillary before the shrinking process at time t=0. The beam parameters were hold constant at an electron high tension of 4 kV, beam current of 119 pA, a magnification of 200 kX times and a working distance of 5.1 mm. Analysis of the black hole in the middle with ImageJ resulted in a diameter (d) of 42 nm. (b) After two minutes of constant imaging under the SEM beam first contraction of the diameter can be observed. Analysis of the black pore resulted in an inner diameter of 33 nm. (c) Past 4 minutes after starting to image the nanopore had further shrunken reaching a diameter of 23 nm. (d) A final diameter of 11 nm was reached after 6 minutes. The beam parameters were held constant over the whole shrinking process. Note that the process of shrinking is uniform from all sides.

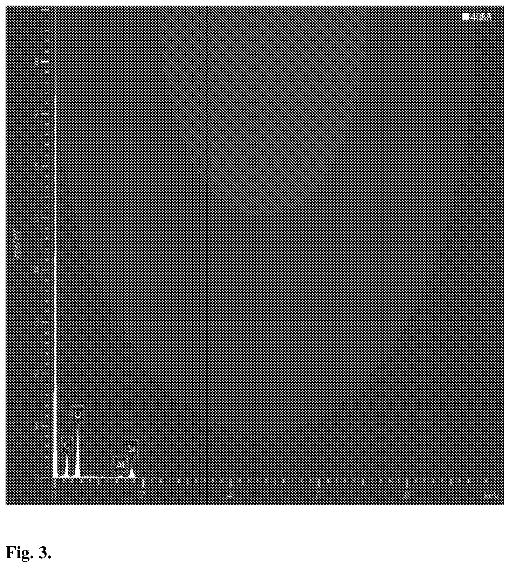

**Fig. 3.** EDX spectrum recorded for the nanocapillary shown in Figure 1 (a). Working distance was 8.1 mm, with an acceleration potential of 3 kV, a beam current of 119 pA and a magnification of 153 kX. The areas of the different peaks were calculated to deduce the relative chemical composition of the nanocapillary. The composition before and after the shrinking is displayed in Table S1.

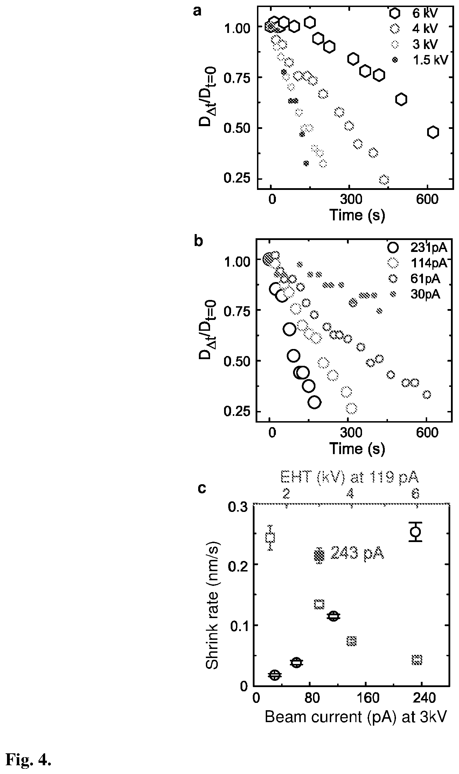

**Fig. 4.** (a) Diameter shrinking over time while irradiated by the SEM beam. The diameter at the different time points was normalized by dividing by the initial diameter at time zero. The shrinking was recorded with a constant beam current of 119 pA but varying beam potential (also called EHT for extra-high tension) between 1.5 kV to 6 kV. Interestingly the smaller the electron high tension is, the faster the shrinking of the diameter takes place. While the diameter shrinks to 0.5 of the initial diameter after 150 seconds at 1.5 kV it takes over 600 seconds to reach this ratio at 6 kV. (b) In contrast to the Fig. 4 (a) the beam potential was held constant at 3 kV and the beam current was varied from 30 to 231 pA. For a beam current of 231 pA the nanocapillary shrinks to 25 % of its initial size after only 150 seconds while a low beam current of 30 pA it reaches only 75% of its original diameter after more than 450 seconds. (c) Shrink rate of the nanocapillary as a function of the beam parameters such as beam current (black lower abscissa) and electron high tension (blue upper abscissa). The shrink rate was calculated from the previous diameter-time dependence in Fig 4. (a) and (b) by calculating the slope using a linear fit. The shrink rate shows a linear dependence from the beam current (hollow black circles); while a power law becomes apparent for the relation between the shrink rate and the beam potential (hollow blue squares). This is expected from the Kanaya-Okayama formula (equation (1)). The filled blue square at beam potential 3 kV was recorded at a beam current of 243 pA twice as high than the other data points at 119 pA. The doubled shrink rate is in agreement with the linear dependence between the beam current and the shrink rate.

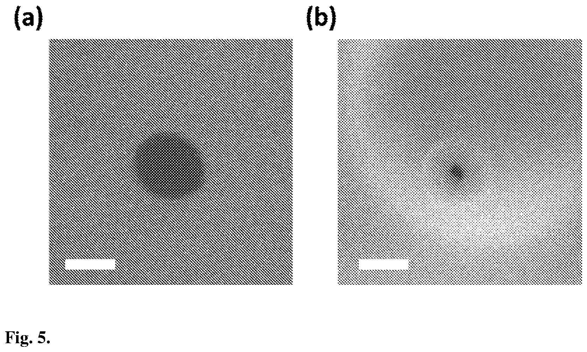

**Fig. 5.** (a) Nanocapillary before starting the shrinking process with a diameter of about 71 nm. The beam potential was 6 kV, the beam current 171 pA, the working distance 5.1 mm, and the magnification was 292 kX. The scale bar represents 50 nm. (b) After 12 minutes of SEM irradiation with constant parameters the nanocapillary had shrunken to a diameter of about 11 nm and was used for DNA translocation experiments. The scale bar represents 50 nm.

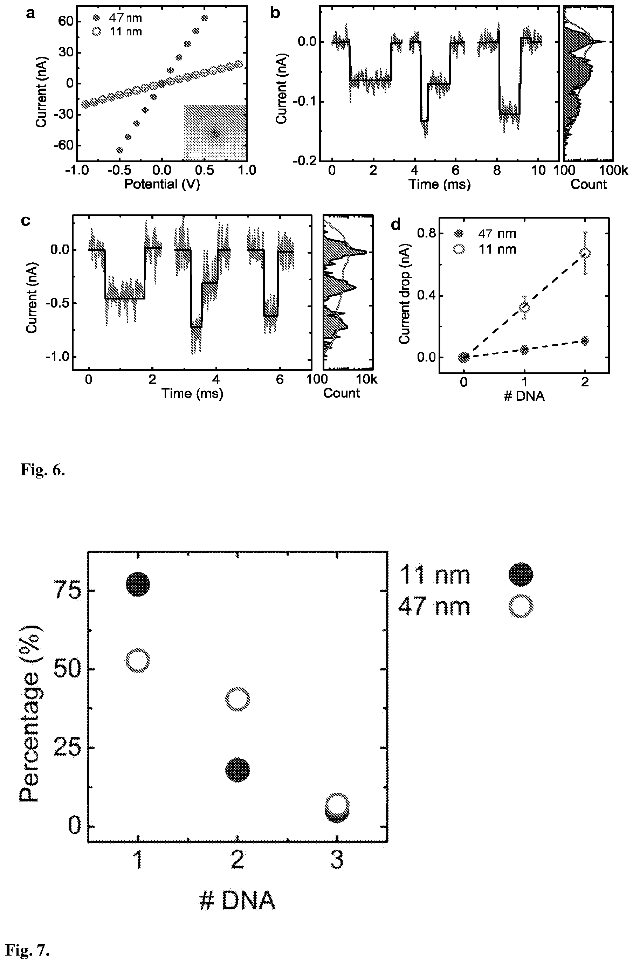

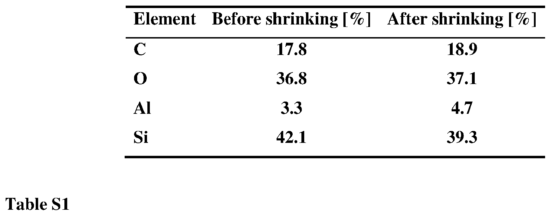

**Fig. 6.** (a) Current in dependence of the applied potential for a nanopore shrunken under an SEM beam to 11 nm and an unmodified nanopore of approximately 47 nm. The KCl solution was 1 M for both nanocapillaries and resulted in a conductance of 21 nS for the shrunken nanocapillary and 128 nS for the unmodified nanocapillary. The inset shows the shrunken nanocapillary with a diameter of around 11 nm. The white scale bar represents 20 nm. (b) Current as a function of time showing the translocation of λ-DNA with three exemplary current drops (blue line) at 0.5 V. The current was normalized to zero to improve comparison between the events. The black line is a CUSUM fit described by Raillon et al. The graph in the right is the histogram of the current traces (blue line) and the fits (black line) for hundreds of events. The histogram shows the quantification of the events with the biggest peak at zero current representing zero DNA strands inside the open nanopore. The blue line is generated from the raw current signal, while the black line results from the CUSUM fit[37]. The second and third peaks around 49 pA and 109 pA are caused by one and two DNA molecules residing inside the nanocapillary, respectively. (c) Three exemplary current traces (green line) recorded for DNA translocation through the 11 nm big nanocapillary at 0.5 V. The black line is the result from the CUSUM fit. The right graph is a histogram from several hundreds of events similar to the ones displayed on the left side. The green line is generated from the raw current data, while the black line results from the CUSUM fit. (d) Current drop as a function of the number of DNA strands for the nanocapillary with an inner diameter of 11 nm (green hollow circles) and 47 nm (blue circles) when applying 0.5 V. The values were obtained by fitting a Gauss function to the peaks generated from the CUSUM in the histograms of Fig. 6. (b) and (c). With current drops of 323 pA for one DNA molecule for the 11 nm nanocapillary compared to only 49 pA for the 47 nm nanocapillary a clear increase in the current blockage for smaller nanocapillaries can be observed.

**Fig. 7.** Percentage of the different folded states of the DNA inside the shrunken (blue spheres) and unmodified nanocapillary (green circles). When translocating through the shrunken nanocapillary more than 75 % of the events are caused by an unfolded DNA strand while less than 25 % are in folded or multiple folded states (blue spheres at # DNA 2 and 3). In contrast only about 50 % of the DNA is unfolded when translocating through an unmodified and bigger nanocapillary (green circle at 1 DNA strand). While over 35 % of the DNA is translocating in a folded state (green circle at 2 and 3 # DNA).

**Table S1.** Comparison of the chemical composition determined by EDX measurements before and after the shrinking of the glass nanocapillary. The respective nanocapillary before and after shrinking can be seen in Fig. 2. The relative composition percentage of carbon (C) did only increase by 1.1 % (from 17.8% to 18.9%) representing only small increase not able to explain the shrinking from 179 to 93 nm in horizontal and 106 to 29 nm in vertical direction.

The quartz capillaries were purchased with an inner and outer diameter of 0.3 and 0.5 mm (Hilgenberg, Germany). The capillary were pulled with the laser pipette puller P-2000 (Sutter, USA). The pulling parameters were Heat 550, Filament 0, Velocity 50, Deletion 130 and Pull 150 resulting in a single pull after an activated laser for about 1.05 seconds. This resulted in nanocapillaries with a taper length of approximately 4 mm. Detailed description of capillary pulling can be found in previous publications[25,35].

The resulting nanocapillaries from the pull were imaged under a Field Emission Scanning electron Microscope (FESEM or SEM). The Merlin SEM (Zeiss, Germany) did not necessitate the presence of a conducting layer on the glass nanocapillaries when imaging with the in-lens detector. This allowed determining the diameter of every nanocapillary before assembling it into the measuring cell, which was not possible before[25]. SEM imaging was performed under a working distance between 2 and 9 mm, magnifications between 10k and 500k, beam currents between 10 and 5000 pA and acceleration voltages of 0.1 to 100 kV. EDX measurements were also possible, permitting it to measure the chemical composition before and after the shrinking. EDX measurements were performed using the AZtexEnergy software under a working distance of about 8 mm and beam potentials of 3 kV or higher.

The nanocapillaries were assembled into a PDMS cell, whose two reservoirs were only connected by the glass orifice[4]. The bottom of the PDMS cell sealed with a 0.15 mm thick cover glass (Menzel-Glässer, Germany). The reservoirs were filled with a potassium chloride (KCl) solution of 1 mol/L (M), 1 mM Tris and 0.1 mM EDTA buffer at pH 8. The solution was cleared from contaminating particles using an anotop 25 filter (Watman, USA). To remove air bubbles inside the nanocapillary after addition of the buffer solution the PDMS cell was degased inside a desiccator using a vacuum line[35]. Oxygen plasma for minutes did improve this step by rendering the surface hydrophilic.

To apply a potential and measure the ionic current the current amplifier Axopatch 200B was used (Axon Instruments, USA) with a low pass Bessel filter at 10 kHz and a PXI-4461 DAQ card (National Instruments, USA) sampling at a frequency of 100 kHz. The electrodes were made out of chlorinated silver electrodes (Ag/AgCl) which were placed on both sides of the nanocapillary to measure the ionic current through the nanocapillary. The DNA translocation events were recorded and analyzed using a custom written Lab VIEW program and a CUSUM algorithm, respectively[36,37].

The inventors surprisingly found that the diameter of nanocapillaries made out of quartz or borosilicate glass shrinks similar to silicon nanopores when imaged under SEM electron beam[19]. This enables one to reach any desired diameter with nanocapillaries. This has an important impact on many fields such as an increased sensitivity for the resistance pulse technique or on the resolution of the scanning electrochemical microscopy[12,32].

Figure 1 (a) presents a schematic representation of nanocapillary showing the conical shape and the region of the tip, which contains a single nanopore. The shaded circle depicts the area, which is irradiated by the electron beam when imaged by the SEM. The penetration depth of the electron entering the quartz glass made out of mainly $SiO_2$ can be estimated by the Kanaya-Okayama depth penetration formula:

$$R = \frac{0.0276\, A\, U^{1.67}}{Z^{0.89} \rho} \, \mu m. \tag{1}$$

The expression describes the penetration depth R of the electrons in dependence from the beam potential (kV), $U$, the atomic weight (g/mol), $A$, the atomic number, $Z$, and the density of the imaged material ($g/cm^2$), $\rho$. The penetration depth $R$ is depicted in Figure 1 (a) with a black circle. From this equation one can see, that the penetration depth, $R$, increases with higher beam potentials, $U$. Using the penetration depth an electron density, $d_e$, can be defined as:

$$d_e = \frac{N_e}{\frac{1}{2}\pi R^3 4/3}. \qquad (2)$$

$N_e$ represents the number of electrons and $\frac{1}{2}\pi R^3 4/3$ stands for the hypothetical penetration volume represented by a half sphere. Calculating the electron density ($d_e$) once can see that increasing the penetration depth, $R$, by having higher beam potentials decreases $d_e$. Increasing the beam current (number of electrons per time) increases the number of electrons and therefore augments the electron density. If one assumes a linear dependence between the electron density and the energy density it can be predicted that the energy density and hence the thermal heating will increase with higher beam currents or with smaller beam potentials. Figure 1 (b) shows a side view of a nanocapillary with a vertical inner diameter of about 175 nm at a working distance of 3.3 mm, a beam current of 171 pA and a beam potential of 3.0 kV. While holding the magnification constant at 196 kX and imaging the nanocapillary for 14 minutes the diameter shrinks approximately to 83 nm (see Figure 1 (c)). To facilitate determination of the inner diameter, nanocapillaries were aligned concentric to the electron beam. This enabled precise determination of its inner diameter (see Figure 2(a) to (d)). The image sequence shows the constant shrinking of the nanocapillary under a beam potential of 4 kV, a beam current of 119 pA, a working distance of 5.1 mm and a magnification of 200 kX. The nanocapillary at time zero in Figure 2 (a) has an inner diameter of 42 nm, which constantly shrinks reaching 33 nm after 2 minutes (Figure 2 (b)), 23 nm after 4 minutes (Figure 2 (c)) and 11 nm after 6 minutes (Figure 2 (d)). Carbon deposition was ruled out by Energy dispersive X-ray spectroscopy (EDX). EDX measurements have been performed before and after shrinking of the quartz nanocapillaries to measure the increase in the carbon composition. Similar measurements were already done by Prabhu et al. who investigated shrinking in silicon nitride nanopores[19]. Spectra like in Figure 3 were recorded using the AZtecEnergy software package, while having a working distance of about 8 mm and an acceleration potential of 3 kV. The software measured the chemical composition of our sample and allowed to quantify the relative amount of carbon on the nanocapillary before and after the shrinking. Figure 3 shows the spectrum before shrinking the nanocapillary, showing typical elements composing a quartz glass like oxygen (O), aluminum (Al), silicon (Si) and carbon (C). After 16 minutes of SEM irradiation the nanocapillary had shrunken from a vertical high of 106 nm to approximately 29 nm. The EDX measurement before and after the shrinking showed an increase of the carbon composition from 17.8 to only 18.9 % (see Table S1). This increase of only 1.1 % can be excluded to play an important factor in the shrinking process, which decreased the horizontal diameter from 179 to 93 nm and the horizontal diameter from 106 to 29 nm. The other chemical elements did also not show notable increase in the composition.

To compare the effect of different parameters such as the beam potential or beam current a normalized diameter unit was chosen. For that the diameter value, D, was divided at time $\Delta t$ by the initial diameter value, Do, at time point zero (t=0). Figure 4 (a) shows the shrinking in the normalized diameter when imaged at different beam potentials ranging from 1.5 kV to 6 kV and a constant beam current of 119 pA. One can see that at lower potentials the shrinking process happens much quicker reaching 25 % of the initial size already after 150 seconds at 1.5 kV. At higher beam potentials the shrinking process manifests much slower reaching only 50 % after 600 seconds at 6 kV. The opposite behavior can be seen when the beam potential was held constant at 3 kV and the beam current was changed. In this case lower beam current values induce a slow shrinking and high values and fast shrinking (see Figure 4 (b)). While a beam of 30 pA causes the nanocapillary to shrink to only 75% after 450 seconds the nanopores shrinks to 25% of its initial size after only 150 seconds when imaged at 231 pA. Figure 2 (a) and (b) support our prediction, which anticipates faster shrinking with increasing beam currents but lower shrink rates with increasing beam potentials. To quantify the shrink rate the diameter changes were fitted with a linear function and the resulting slope was plotted in dependence of the beam current and the beam potential (see Figure 4 (c)). The shrink rate as a function of the beam current shows a linear dependence with a rate ranging from about 0.02 nm/s at 30 pA to 0.25 nm/s at 231 pA (black circles in Figure 4 (c)). In contrast, the shrink rate shows power law dependence when plotted as a function of the beam potential (blue squares in Figure 4 (c)). Both dependences agree with our model. The number of electrons increases linearly with the beam current, augmenting the energy density inside the glass. This causes thermal heating of the nanocapillary and its diameter shrinking due to the surface stress (see equation (2)). But when the beam potential is increased the penetration depth of the electron entering the glass is increased. This diminishes the energy density, resulting in less heating and therefore smaller shrink rates with increasing beam potentials (see equation (1) and (2)).

The ability to shrink nanocapillaries to any size has wide applications. One of them is the resistive pulse technique, which profits from a smaller nanocapillary with an increase in the signal amplitude[12,38]. To prove this a nanocapillary was shrunken to a diameter of 11 nm and incorporated into a PDMS cell (see Figure 5 (a) and (b)). The nanocapillary was filled with a 1 M KCl buffer solution and the current was measured of a range of potentials (see green circles in Figure 6 (a)). Fitting this IV-curve with a linear function reveals the slope which gives a conductance of 21 nS.

To illustrate the effect of the shrinking an IV-curve was also recorded with an unmodified nanocapillary (see blue circles in Figure 6 (a)). The unshrunken nanocapillary with an approximated diameter of 47 nm showed a higher conductance of 128 nS than the 11 nm nanocapillary with only 21 nS. Next, a 0.5 $\mu$g/$\mu$L $\lambda$-DNA solution of the same ionic strength was added to the reservoir in front of the shrunken and unmodified nanocapillary. A positive potential was applied to the electrode inside the nanocapillary causing the $\lambda$-DNA to translocate into the nanocapillary and reduce the ionic current[39]. Figure 6 (b) and (c) show exemplary current traces recorded at 0.5 V. It is characterized by quantized decreasing steps, revealing the number of DNA strands inside the unmodified (blue trace) and shrunken nanocapillary (green trace)[25,40]. The black line is a fit by a previously described CUSUM algorithm by Raillon et al. The algorithm allows the analysis of noisy data by generating better resolved histograms[37]. This becomes visible when looking at the histogram in Figure 6 (c) where the CUSUM generates peaks (black line) which are better pronounced than the peaks from the raw data (green line). The first and biggest peak represents the open pore current and is normalized to zero pA to better compare events in case of drifting baseline current. The second and third peak represent one or two DNA strands inside the nanocapillary[25,40]. The peaks from the CUSUM fit in the histograms were fitted with Gauss functions to determine their position and plotted in Figure 6 (d) against the number of DNA strands. As predicted the current decreases due to DNA strands inside the nanocapillary are much bigger when translocating through a smaller nanocapillary than a bigger nanocapillary. While a single DNA strand causes reduction of 49 pA in the unmodified nanocapillary the DNA inside the shrunken nanocapillary (11 nm diameter) generates a more than six time stronger decrease of 323 pA. This expected increase in the signal amplitude permits detecting smaller molecules such as single stranded DNA, RNA or even proteins.

The size effect of the nanocapillary has also been investigated on the folding ratio of the translocating DNA. For this the number of data points within each folded state (one, two and three or more DNA strands inside the nanocapillary) was summed up and divided by the total number of states. Figure 7. shows this distribution of the folding states. When translocating through the small, shrunken nanocapillary the DNA prefers an unfolded state with over 75% of all current decreases being due to one DNA strand inside the constriction (blue sphere at one # DNA). Less than 25 % are caused by folded DNA strands (blue spheres at 2 and 3 # DNA molecules). In contrast, when the DNA moves through an unmodified, big nanocapillary (about 47 nm diameter) more than 37 % of the events are caused by two DNA strands compared to only 20 % when using a shrunken nanocapillary. In consequence fewer events are caused by unfolded DNA inside the nanocapillary making less than 55 % of all possible configurations (see green circle at 1 # DNA). This finding shows that smaller nanocapillaries favor unfolded over folded DNA when translocating as shown for similar systems like solid-state nanopores in silicon membranes[40].

Smaller diameter will increase the resolution of scanning electrochemical microscopy or surface near-field optical microscopy, which are both based on conical glass capillaries. Also, 3D and 2D printing resolution could benefit from small conical pores.

It has been shown that nanometer-sized orifices in quartz and borosilicate glass can be reshaped using an ordinary scanning electron microscope. The shrinking of the nanocapillary occurs within minutes which allow stopping the process at any desired size ranging from 100 to a few nanometers. The shrinking process was explained with a model based on the penetration depth by Kanaya-Okayama. The model predicts a linear dependence of the shrinking rate from the beam current and a power law dependence for the beam potential. This was shown experimentally for various beam currents and beam potentials. This finding enables to fine-control the shrinking by accelerating or decelerating it, permitting to reach small diameters within seconds or switch to slow and well controlled shrink rate if desired. Interesting avenues to pursue include testing the effect of different pipette shapes and SEM instruments on the shrinking behavior. Further, it has been shown that shrinking the inner diameter of nanocapillaries increases the signal amplitude caused by the translocation of DNA. In the future this will make it possible to detect smaller molecules like RNA or proteins translocating through nanocapillaries. Besides improving the resistive pulse technique it will also enhance other techniques like SECM, SNOM, SICM, 2D or 3D-printing.

## REFERENCES

(1) Coulter, W. H. Means for counting particles suspended in a fluid, 1953.

(2) Steinbock, L. J.; Stober, G.; Keyser, U. F. Biosens. Bioelectron. 2009, 24, 2423-2427.

(3) Astier, Y.; Braha, O.; Bayley, H. J. Am. Chem. Soc. 2006, 128, 1705-1710.

(4) Steinbock, L. J.; Lucas, A.; Otto, O.; Keyser, U. F. Electrophoresis 2012, 33, 3480-3487.

(5) Kasianowicz, J. J.; Brandin, E.; Branton, D.; Deamer, D. W. Proc. Natl. Acad. Sci. U. S. A. 1996, 93, 13770-13773.

(6) Kim, M. J.; Wanunu, M.; Bell, D. C.; Meller, A. Adv. Mater. 2006, 18, 3149-3153.

(7) Chansin, G. a. T.; Hong, J.; Dusting, J.; DeMello, A. J.; Albrecht, T.; Edel, J. B. Small 2011, 7,2736-2741.

(8) Kox, R.; Chen, C.; Maes, G.; Lagae, L.; Borghs, G. Nanotechnology 2009, 20, 115302.

(9) Kim, M. J.; McNally, B.; Murata, K.; Meller, A. Nanotechnology 2007, 18, 205302.

(10) Nagoshi, K.; Honda, J.; Sakaue, H.; Takahagi, T.; Suzuki, H. Rev. Sci. Instrum. 2009, 80, 125102.

(11) Shin, J. W.; Lee, J. Y.; Lee, D. U.; Oh, D. H.; Kim, D. H.; Kim, T. W.; Cho, W. J.; Jin, S. Nanotechnology 2009, 20, 075703.

(12) Kowalczyk, S. W.; Grosberg, A. Y.; Rabin, Y.; Dekker, C. Nanotechnology 2011, 22, 315101.

(13) WO/2004/078,640, W. P.; 2004. *patentscope.wipo.int.*

(14) Storm, A. J.; Chen, J. H.; Ling, X. S.; Zandbergen, H. W.; Dekker, C. Nat. Mater. 2003, 2, 537-540.

(15) Danelon, C.; Santschi, C.; Brugger, J.; Vogel, H. Langmuir 2006, 22, 10711-10715.

(16) Lo, C. J.; Aref, T.; Bezryadin, A. Nanotechnology 2006, 17, 3264-3267.

(17) Chang, H.; Iqbal, S. M.; Stach, E. a.; King, A. H.; Zaluzec, N. J.; Bashir, R. Appl. Phys. Lett. 2006, 88, 103109.

(18) Asghar, W.; Ilyas, A.; Billo, J. A.; Iqbal, S. M. Nanoscale Res. Lett. 2011, 6, 372.

(19) Prabhu, A. S.; Freedman, K. J.; Robertson, J. W. F.; Nikolov, Z.; Kasianowicz, J. J.; Kim, M. J. Nanotechnology 2011, 22, 425302.

(20) Radenovic, A.; Trepagnier, E.; Csencsits, R.; Downing, K. H.; Liphardt, J. Appl. Phys. Lett. 2008, 93, 183101.

(21) Zhang, W. M.; Wang, Y. G.; Li, J.; Xue, J. M.; Ji, H.; Ouyang, Q.; Xu, J.; Zhang, Y. Appl. Phys. Lett. 2007, 90, 163102.

(22) Ayub, M.; Ivanov, A.; Hong, J.; Kuhn, P.; Instuli, E.; Edel, J. B.; Albrecht, T. J. Physics. Condens. Matter 2010, 22, 454128.

(23) Chen, P.; Gu, J.; Brandin, E.; Kim, Y.-R. Y. R.; Wang, Q.; Branton, D.; Mitsui, T.; Farmer, D. B.; Golovchenko, J.; Gordon, R. G. Nano Lett. 2004, 4, 2293-2298.

(24) Kim, Y.-R.; Lee, I.-H.; Min, J.; Kim, A.-G.; Kim, S.; Kim, K.; Namkoong, K.; Ko, C. Biosens. Bioelectron. 2007, 22, 2926-2931.

(25) Steinbock, L. J.; Otto, O.; Chimerel, C.; Gornall, J.; Keyser, U. F. Nano Lett. 2010, 10, 2493-2497.

(26) Shao, Y.; Mirkin, M. J. Am. Chem. Soc. 1997, 119, 8103-8104.

(27) Steinbock, L. J.; Otto, O.; Skarstam, D. R.; Jahn, S.; Chimerel, C.; Gornall, J. L.; Keyser, U. F. J. Phys. Condens. Matter 2010, 22, 454113.

(28) Gao, C.; Ding, S.; Tan, Q.; Gu, L.-Q. Anal. Chem. 2009, 81, 80-86.

(29) Schibel, A. E. P.; Edwards, T.; Kawano, R.; Lan, W.; White, H. S. Anal. Chem. 2010, 82, 7259-7266.

(30) Platt, M.; Willmott, G. R.; Lee, G. U. Small 2012, 8, 2436-2444.

(31) Wanunu, M.; Dadosh, T.; Ray, V.; Jin, J.; McReynolds, L.; Drndic, M. Nat. Nanotechnol. 2010, 5, 807-814.

(32) Takahashi, Y.; Shevchuk, A. I.; Novak, P.; Babakinejad, B.; Macpherson, J.; Unwin, P. R.; Shiku, H.; Gorelik, J.; Klenerman, D.; Korchev, Y. E.; Matsue, T. Proc. Natl. Acad. Sci. U. S. A. 2012, 109, 11540-11545.

(33) Gornall, J. L.; Mahendran, K. R.; Pambos, O. J.; Steinbock, L. J.; Otto, O.; Chimerel, C.; Winterhalter, M.; Keyser, U. F. Nano Lett. 2011, 11, 3334-3340.

(34) Hernandez-Ainsa, S.; Muus, C.; Bell, N. a W.; Steinbock, L. J.; Thacker, V. V; Keyser, U. F. Analyst 2013, 138, 104-106.

(35) Steinbock, L. J.; Keyser, U. F. In Methods in Molecular Biology; Gracheva, M. E., Ed.; Springer Science: Totowa, NJ, 2012; Vol. 870, pp. 135-145.

(36) Raillon, C.; Cousin, P.; Traversi, F.; Garcia-Cordero, E.; Hernandez, N.; Radenovic, A. Nano Lett. 2012, 12, 1157-1164.

(37) Raillon, C.; Granjon, P.; Graf, M.; Steinbock, L. J.; Radenovic, A. Nanoscale 2012, 4, 4916-4924.

(38) Willmott, G. R.; Smith, B. G. Nanotechnology 2012, 23, 088001.

(39) Thacker, V. V; Ghosal, S.; Hernandez-Ainsa, S.; Bell, N. A. W.; Keyser, U. F. Appl. Phys. Lett. 2012, 101, 223704.

(40) Li, J.; Gershow, M.; Stein, D.; Brandin, E.; Golovchenko, J. A. Nat. Mater. 2003, 2, 611-615.

**Claims**

1. Process for modifying an orifice of an uncoated glass nanocapillary, the glass nanocapillary being a glass cone comprising an orifice at its tip, said process comprising a step where an initial orifice of a first size is shrinked by electron radiation emitted by a Scanning Electron Microscope (SEM) applied to a tip of the glass nanocapillary defining said orifice, and wherein an electron beam of the Scanning Electron Microscope (SEM) is used to simultaneously act as a heating source applied to the tip of the glass nanocapillary as well as a means to image the orifice.

2. Process according to the previous claim, wherein a shrink rate of the orifice is fine-tuned by changing a current, magnification or acceleration potential of the radiation.

3. Process according to anyone of the previous claims, further comprising imaging of the orifice with the Scanning electron microscope in order to see the shrinking process and stop at a desired size.

4. Process according to anyone of the previous claims, wherein a shrinking rate is changed by adjusting a beam current, magnification or an electron acceleration voltage of the electron radiation.

**Patentansprüche**

1. Prozess zum Modifizieren einer Öffnung einer nichtbeschichteten Glasnanokapillare, wobei die Glasnanokapillare ein Glaskegel ist, der eine Öffnung an seiner Spitze umfasst, wobei der Prozess einen Schritt umfasst, bei dem eine anfängliche Öffnung einer ersten Größe durch Elektronenstrahlung verkleinert wird, die durch ein Rasterelektronenmikroskop (SEM: Scanning Electron Microscope) emittiert wird, das auf eine Spitze der Glasnanokapillare angewandt wird, die die Öffnung definiert, und wobei ein Elektronenstrahl des Rasterelektronenmikroskops (SEM) verwendet wird, um gleichzeitig als eine Heizquelle, die auf die Stütze der Glasnanokapillare angewandt wird, als auch ein Mittel zur bildlichen Erfassung der Öffnung zu dienen.

2. Prozess nach dem vorhergehenden Anspruch, wobei eine Verkleinerungsrate der Öffnung durch Ändern eines Stroms, einer Vergrößerung oder eines Beschleunigungspotentials der Strahlung feinabgestimmt wird.

3. Prozess nach einem der vorhergehenden Ansprüche, das ferner bildliches Erfassen der Öffnung mit dem Rasterelektronenmikroskop umfasst, um den Verkleinerungsprozess zu beobachten und bei einer gewünschten Größe zu stoppen.

**4.** Prozess nach einem der vorhergehenden Ansprüche, wobei eine Verkleinerungsrate durch Anpassen eines Strahlstroms, einer Vergrößerung oder einer Elektronenbeschleunigungsspannung der Elektronenstrahlung geändert wird.

**Revendications**

**1.** Procédé pour la modification d'un orifice d'un nanocapillaire de verre non revêtu, le nanocapillaire de verre étant un cône de verre comprenant un orifice à son sommet, ledit procédé comprenant une étape où un orifice initial d'une première taille est rétréci par un rayonnement d'électrons émis par un Microscope Électronique à Balayage (MEB) appliqué à un sommet du nanocapillaire de verre définissant ledit orifice, et un faisceau d'électrons du Microscope Électronique à Balayage (MEB) étant utilisé pour agir simultanément en tant qu'une source de chaleur appliquée au sommet du nanocapillaire de verre ainsi qu'en tant qu'un moyen pour visualiser l'orifice.

**2.** Procédé selon la revendication précédente, une vitesse de rétrécissement de l'orifice étant affinée en changeant un courant, un agrandissement ou un potentiel d'accélération du rayonnement.

**3.** Procédé selon l'une quelconque des revendications précédentes, comprenant en outre la visualisation de l'orifice avec le microscope électronique à balayage afin de voir le procédé de rétrécissement et de stopper à une taille souhaitée.

**4.** Procédé selon l'une quelconque des revendications précédentes, une vitesse de rétrécissement étant changée par l'ajustement d'un courant de faisceau, d'un agrandissement ou d'un voltage d'accélération d'électrons du rayonnement d'électrons.

**Fig. 1.**

**Fig. 2.**

**Fig. 3.**

**Fig. 4.**

(a)

(b)

**Fig. 5.**

Fig. 6.

Fig. 7.

| Element | Before shrinking [%] | After shrinking [%] |
|---------|---------------------|---------------------|
| C | 17.8 | 18.9 |
| O | 36.8 | 37.1 |
| Al | 3.3 | 4.7 |
| Si | 42.1 | 39.3 |

**Table S1**

## REFERENCES CITED IN THE DESCRIPTION

**Patent documents cited in the description**

- WO 2004078640 A

- WO 0034810 A

**Non-patent literature cited in the description**

- **BO ZHANG et al.** A Silica Nanochannel and Its Applications in Sensing and Molecular Transport. *ANALYTICAL CHEMISTRY,* 01 July 2009, vol. 81 (13), ISSN 0003-2700, 5541-5548
- Probing DNA with micro-and nanocapillaries and optical tweezers. **STEINBOCK L J et al.** JOURNAL OF PHYSICS: CONDENSED MATTER. INSTITUTE OF PHYSICS PUBLISHING, 29 October 2010, vol. 22, 454113
- **ANMIV S PRABHU et al.** SEM-induced shrinking of solid-state nanopores for single molecule detection. *NANOTECHNOLOGY, IOP, BRISTOL,* 22 September 2011, vol. 22, 425302
- **COULTER, W. H.** *Means for counting particles suspended in a fluid,* 1953
- **STEINBOCK, L. J. ; STOBER, G. ; KEYSER, U. F.** *Biosens. Bioelectron.,* 2009, vol. 24, 2423-2427
- **ASTIER, Y. ; BRAHA, O. ; BAYLEY, H.** *J. Am. Chem. Soc.,* 2006, vol. 128, 1705-1710
- **STEINBOCK, L. J. ; LUCAS, A. ; OTTO, O. ; KEYSER, U. F.** *Electrophoresis,* 2012, vol. 33, 3480-3487
- **KASIANOWICZ, J. J. ; BRANDIN, E. ; BRANTON, D. ; DEAMER, D. W.** *Proc. Natl. Acad. Sci. U. S. A.,* 1996, vol. 93, 13770-13773
- **KIM, M. J. ; WANUNU, M. ; BELL, D. C.** *Meller, A. Adv. Mater.,* 2006, vol. 18, 3149-3153
- **CHANSIN, G. A. T. ; HONG, J. ; DUSTING, J. ; DEMELLO, A. J. ; ALBRECHT, T. ; EDEL, J. B.** *Small,* 2011, vol. 7, 2736-2741
- **KOX, R. ; CHEN, C. ; MAES, G. ; LAGAE, L. ; BORGHS, G.** *Nanotechnology,* 2009, vol. 20, 115302
- **KIM, M. J. ; MCNALLY, B. ; MURATA, K. ; MELLER, A.** *Nanotechnology,* 2007, vol. 18, 205302
- **NAGOSHI, K. ; HONDA, J. ; SAKAUE, H. ; TAKAHAGI, T. ; SUZUKI, H.** *Rev. Sci. Instrum.,* 2009, vol. 80, 125102
- **SHIN, J. W. ; LEE, J. Y. ; LEE, D. U. ; OH, D. H. ; KIM, D. H. ; KIM, T. W. ; CHO, W. J. ; JIN, S.** *Nanotechnology,* 2009, vol. 20, 075703

- **KOWALCZYK, S. W. ; GROSBERG, A. Y. ; RABIN, Y. ; DEKKER, C.** *Nanotechnology,* 2011, vol. 22, 315101
- **STORM, A. J. ; CHEN, J. H. ; LING, X. S. ; ZANDBERGEN, H. W. ; DEKKER, C.** *Nat. Mater.,* 2003, vol. 2, 537-540
- **DANELON, C. ; SANTSCHI, C. ; BRUGGER, J. ; VOGEL, H.** *Langmuir,* 2006, vol. 22, 10711-10715
- **LO, C. J. ; AREF, T. ; BEZRYADIN, A.** *Nanotechnology,* 2006, vol. 17, 3264-3267
- **CHANG, H. ; IQBAL, S. M. ; STACH, E. A. ; KING, A. H. ; ZALUZEC, N. J. ; BASHIR, R.** *Appl. Phys. Lett.,* 2006, vol. 88, 103109
- **ASGHAR, W. ; ILYAS, A. ; BILLO, J. A. ; IQBAL, S. M.** *Nanoscale Res. Lett.,* 2011, vol. 6, 372
- **PRABHU, A. S. ; FREEDMAN, K. J. ; ROBERTSON, J. W. F. ; NIKOLOV, Z. ; KASIANOWICZ, J. J. ; KIM, M. J.** *Nanotechnology,* 2011, vol. 22, 425302
- **RADENOVIC, A. ; TREPAGNIER, E. ; CSENCSITS, R. ; DOWNING, K. H. ; LIPHARDT, J.** *Appl. Phys. Lett.,* 2008, vol. 93, 183101
- **ZHANG, W. M. ; WANG, Y. G. ; LI, J. ; XUE, J. M. ; JI, H. ; OUYANG, Q. ; XU, J. ; ZHANG, Y.** *Appl. Phys. Lett.,* 2007, vol. 90, 163102
- **AYUB, M. ; IVANOV, A. ; HONG, J. ; KUHN, P. ; INSTULI, E. ; EDEL, J. B. ; ALBRECHT, T.** *J. Physics. Condens. Matter,* 2010, vol. 22, 454128
- **CHEN, P. ; GU, J. ; BRANDIN, E. ; KIM, Y.-R. Y. R ; WANG, Q. ; BRANTON, D. ; MITSUI, T. ; FARMER, D. B. ; GOLOVCHENKO, J. ; GORDON, R. G.** *Nano Lett.,* 2004, vol. 4, 2293-2298
- **KIM, Y.-R. ; LEE, I.-H. ; MIN, J. ; KIM, A.-G. ; KIM, S. ; KIM, K. ; NAMKOONG, K. ; KO, C.** *Biosens. Bioelectron.,* 2007, vol. 22, 2926-2931
- **STEINBOCK, L. J. ; OTTO, O. ; CHIMEREL, C. ; GORNALL, J. ; KEYSER, U. F.** *Nano Lett.,* 2010, vol. 10, 2493-2497
- **SHAO, Y. ; MIRKIN, M.** *J. Am. Chem. Soc.,* 1997, vol. 119, 8103-8104

- **STEINBOCK, L. J. ; OTTO, O. ; SKARSTAM, D. R. ; JAHN, S. ; CHIMEREL, C. ; GORNALL, J. L. ; KEYSER, U. F.** *J. Phys. Condens. Matter,* 2010, vol. 22, 454113
- **GAO, C. ; DING, S. ; TAN, Q. ; GU, L.-Q.** *Anal. Chem.,* 2009, vol. 81, 80-86
- **SCHIBEL, A. E. P ; EDWARDS, T. ; KAWANO, R. ; LAN, W. ; WHITE, H. S.** *Anal. Chem.,* 2010, vol. 82, 7259-7266
- **PLATT, M. ; WILLMOTT, G. R. ; LEE, G. U.** *Small,* 2012, vol. 8, 2436-2444
- **WANUNU, M. ; DADOSH, T. ; RAY, V. ; JIN, J. ; MCREYNOLDS, L. ; DRNDIC, M.** *Nat. Nanotechnol.,* 2010, vol. 5, 807-814
- **TAKAHASHI, Y. ; SHEVCHUK, A. I. ; NOVAK, P. ; BABAKINEJAD, B. ; MACPHERSON, J. ; UNWIN, P. R. ; SHIKU, H. ; GORELIK, J. ; KLENERMAN, D. ; KORCHEV, Y. E.** *Proc. Natl. Acad. Sci. U. S. A.,* 2012, vol. 109, 11540-11545
- **GORNALL, J. L. ; MAHENDRAN, K. R. ; PAMBOS, O. J. ; STEINBOCK, L. J. ; OTTO, O. ; CHIMEREL, C. ; WINTERHALTER, M. ; KEYSER, U. F.** *Nano Lett.,* 2011, vol. 11, 3334-3340
- **HERNANDEZ-AINSA, S. ; MUUS, C. ; BELL, N. A W. ; STEINBOCK, L. J. ; THACKER, V. V ; KEYSER, U. F.** *Analyst,* 2013, vol. 138, 104-106
- **STEINBOCK, L. J. ; KEYSER, U. F.** Methods in Molecular Biology. Springer Science, 2012, vol. 870, 135-145
- **RAILLON, C. ; COUSIN, P. ; TRAVERSI, F. ; GARCIA-CORDERO, E. ; HERNANDEZ, N. ; RADENOVIC, A.** *Nano Lett.,* 2012, vol. 12, 1157-1164
- **RAILLON, C. ; GRANJON, P. ; GRAF, M. ; STEINBOCK, L. J. ; RADENOVIC, A.** *Nanoscale,* 2012, vol. 4, 4916-4924
- **WILLMOTT, G. R. ; SMITH, B. G.** *Nanotechnology,* 2012, vol. 23, 088001
- **THACKER, V. V ; GHOSAL, S. ; HERNANDEZ-AINSA, S. ; BELL, N. A. W. ; KEYSER, U. F.** *Appl. Phys. Lett.,* 2012, vol. 101, 223704
- **LI, J. ; GERSHOW, M. ; STEIN, D ; BRANDIN, E ; GOLOVCHENKO, J. A.** *Nat. Mater.,* 2003, vol. 2, 611-615